# EUROPEAN PATENT SPECIFICATION

(11) **EP 0 970 203 B1**
(45) Date of publication and mention of the grant of the patent: **23.05.2007**
(21) Application number: 97909219.4
(22) Date of filing: 22.08.1997
(51) Int. Cl.: C12N 15/12, C07K 14/47, C12Q 1/68, G01N 33/50

(54) **AGENTS FOR PRE-SYMPTOMATIC DETECTION AND THERAPEUTIC TARGETING OF ALZHEIMER'S DISEASE IN HUMANS**
SUBSTANZEN FÜR DIE PRÄSYMPTOMATISCHE ERKENNUNG UND ZIELGERICHTETE THERAPIE DER ALZHEIMER-KRANKHEIT BEIM MENSCHEN.
AGENTS POUR LA DETECTION PRE-SYMPTOMATIQUE ET LE CIBLAGE THERAPEUTIQUE DE LA MALADIE D'ALZHEIMER CHEZ L'HOMME

(30) Priority: 22.08.1996 CA 2183901
(43) Date of publication of application: 12.01.2000
(73) Proprietor: BERGMANN, Johanna, E., D-22587 Hamburg (DE); Preddie, Rick, E., 22587 Hamburg (DE)
(72) Inventor: BERGMANN, Johanna, E., D-22587 Hamburg (DE); Preddie, Rick, E., 22587 Hamburg (DE)
(86) International application number: PCT/EP1997/004599
(87) International publication number: WO 1998/007850

(56) References cited:
- WO-A-92/13069
- WO-A-93/07296
- WO-A-94/00569
- WO-A-94/13798
- CA-A- 2 126 787
- BERGMANN JE ET AL: "'ALZAS', a protein found in brain and blood of humans with Alzheimer's Disease (AD) but not in normal humans, appears to be the causative biochemical factor of all forms of AD" NEUROBIOLOGY OF AGING, vol. 17, no. 4 suppl., 1996, pages s14-s15, XP002067114
- LEVY E ET AL: "Mutation of the Alzheimer's Disease Amyloid gene in hereditary cerebral hemorrhage, Dutch type" SCIENCE, vol. 248, 1990, US, pages 1124-1126, XP002067115

### SEQUENCE LISTING

(1) GENERAL INFORMATION:
   (i) APPLICANT: Bergmann, Johanna &
      Preddie, Rick
   (ii) TITLE OF INVENTION: AGENTS FOR PRESYMPTOMATIC DIAGNOSIS AND THERAPEUTIC TARGETING IN HUMAN ALZHEIMER'S DISEASE AND DOWN SYNDROME
   (iii) NUMBER OF SEQUENCES:
   (iv) CORRESPONDENCE ADDRESS:
      (A) ADDRESSEE: DR. J. BERGMANN
      (B) STREET: MORIKESTR. 22
      (C) CITY: HAMBURG
      (D) STATE:
      (E) COUNTRY: GERMANY
      (F) ZIP: 22587
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #125
   (vi) CURRENT APPLICATION DATA:
      (A) APPLICATION NUMBER: PCT
      (B) FILING DATE:
      (C) CLASSIFICATION:
   (viii) ATTORNEY/AGENT INFORMATION:
      (A) NAME: NONE
      (B) REGISTRATION NUMBER:
   (ix) TELECOMMUNICATION INFORMATION:
      (A) TELEPHONE: (4940) 862-576
      (B) TELEFAX: (4940) 862-596
(2) INFORMATION FOR SEQ ID NO:11:
   (i) SEQUENCE CHARACTERISTICS:
      (A) LENGTH: 241 base pairs
      (B) TYPE: nucleic acid
      (D) TOPOLOGY: linear
      (C) STRANDNESS: single
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: alzas
   (xi) SEQUENCE DESCRIPTION: SEQ ID NO:11:
(2) FORMATION FOR SEQ ID NO:12:
   (i) SEQUENCE CHARACTERISTICS:
      (A) LENGTH: 79 amino acids
      (B) TYPE: amino acid
      (D) TOPOLOGY: linear
   (ii) MOLECULE TYPE: protein
   (iii) HYPOTHETICAL: NO
   (vi) ORIGINAL SOURCE:
      (A) ORGANISM : Homo sapiens
   (vii) IMMEDIATE SOURCE:
      (B) CLONE: ALZASp
   (xi) SEQUENCE DESCRIPTION: SEQ ID NO:12
(2) INFORMATION FOR SEQ ID NO:17:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: PALZ1
   (xi) SEQUENCE DESCRIPTION: SEQ ID NO:17:
   TTGATAATTA AATGTTATAG CATGGACACT GACATTTACA TTTTTTACTT 50
   ATGTTTTTGG TTTTTAAATG ACTCTGCAT 79
(2) INFORMATION FOR SEQ ID NO:18:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: Genomic DNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: PALZ2
   (xi) SEQUENCE DESCRIPTION: SEQ ID NO:18:
      ATTATTATTT GAATAATGAA ATTCATCAGA ACAATTA 37
(2) INFORMATION FOR SEQ ID NO:19:
   (i) SEQUENCE CHARACTERISTICS:
      (A) LENGTH: 68 base pairs
      (B) TYPE: nucleic acid
      (D) TOPOLOGY: linear
      (C) STRANDNESS: single
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE:PALZ3
   (xi) SEQUENCE DESCRIPTION: SEQ ID NO:19:
(2) INFORMATION FOR SEQ ID NO:20:
   (i) SEQUENCE CHARACTERISTICS:
      (A) LENGTH: 59 base pairs
      (B) TYPE: nucleic acid
      (D) TOPOLOGY: linear
      (C) STRANDNESS: single
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE:PALZ4
   (xi) SEQUENCE DESCRIPTION: SEQ ID NO:20:
   ATGCTCATTT TTAAAGGCTT TTATTATTAT TTCTGAAGTA ATGAGTGCAC   50
   ATGGAAAAA                                                59
(2) INFORMATION FOR SEQ ID NO:21:
   (i) SEQUENCE CHARACTERISTICS:
      (A) LENGTH: 38 base pairs
      (B) TYPE: nucleic acid
      (D) TOPOLOGY: linear
      (C) STRANDNESS: single
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE:PALZ5
   (xi) SEQUENCE DESCRIPTION: SEQ ID NO:21:
      TATTCCAGGA ACAAATCCTT GCCAACCTCT CAACCAGG 38
(2) INFORMATION FOR SEQ ID NO:22:
   (i) SEQUENCE CHARACTERISTICS:
      (A) LENGTH: 26 base pairs
      (B) TYPE: nucleic acid
      (D) TOPOLOGY: linear
      (C) STRANDNESS: single
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE:PALZ6
   (xi) SEQUENCE DESCRIPTION: SEQ ID NO:22:
      TAGCATGTAT TTAAATGCAG CAGAAG 26
(2) INFORMATION FOR SEQ ID NO:23:
   (i) SEQUENCE CHARACTERISTICS:
      (A) LENGTH: 56 base pairs
      (B) TYPE: nucleic acid
      (D) TOPOLOGY: linear
      (C) STRANDNESS: single
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE:PALZ7
   (xi) SEQUENCE DESCRIPTION: SEQ ID NO:23:
   GAAGGTTTAA ATATAGGGTA TCATTTTTCT TTAAGAGTCA TTTATCAATT 50
   TTCTTC                                                 56

## Description

### FIELD OF THE INVENTION:

The invention relates to highly specific diagnostic markers which are also highly specific targets for therapeutic reagents which are useful for detecting, preventing and treating Alzheimer's disease . More specifically, the invention relates to two unique proteins encoded on human chromosome 21, within the locus found to be occupied by human APP gene, to analogues and derivatives of these molecules, and to nucleic acid molecules encoding such molecules or influencing its expression. The invention also relates to therapeutic methods involving these molecules.

### BACKGROUND OF THE INVENTION:

Alzheimer's disease ("AD") is a progressive disease of the human central nervous system. It is manifested by dementia in the elderly, by disorientation, loss of memory, difficulty with language, calculation, or visual-spacial skills, and by psychiatric manifestations. It is associated with degenerating neurons in several regions of the brain. All humans with AD develop a considerable number of β amyloid plaques and neurofibrillary tangles composed of highly phosphorylated forms of the microtubule associated protein tau; Alzheimer's disease is reviewed by Price, D.L. et al. (Clin. Neuropharm. 14:S9-S14 (1991)); Pollwein, P. et al. (Nucl. Acids Res. 20:63-68 (1992)); Regland, B. et al. (Med. Hypoth. 38:11-19 (1992)) and Johnson, S.A. (In: Review of Biological Research in Aging, Vol. 4., Rothstein, M. (Ed.), Wiley-Liss, NY, 163-170 (1990)).

The presence in postmortem brains of two proteins, hyperphosphorylated tau (PHF tau) and β-amyloid (Aβ), is the major pathological feature of all forms of human Alzheimer's disease (AD) and provides certain clues to some biochemical events in the disease. Normal tau is a cytoskeleton protein that functions as a microtubule "glue" in neurons, and β-amyloid is a generally neurotoxic polypeptide fragment which is thought to be excised from the widely distributed APP transmembrane protein. PHF tau is present in the CSF of all humans with AD and Aβ is present in CSF and blood platelets of people with AD. Another significant clue to physiological processes which contribute to the etiology of AD is the presence of numerous inflammatory proteins in characteristic Alzheimer's lesions.

### Genes predisposing to AD.

Four genes and a protein, viz. the APP gene on chromosome #21 (St. George Hyslop, P. et al, Science 235:885-890 1987; Goate, A. M. et al, Nature 349:704-706 1991) S182 gene on chromosome #14 (Sherrington, R. et al. Nature 375:754-760 1995), the STM2 gene on chromosome #1 (Levy-Lahad, E. et al. Science 269 : 973-977 1995) and the ApoE4 gene on chromosome #19 (Saunders, A.M. et al., Neurology 43:1467-1472 1993) has been definitely implicated in the etiology of the disease. The neuron specific cytoskeletal protein "tau" which is secreted into spinal fluid, is highly phosphorylated in AD and the latter form is found in quantities, related to the stage of the disease, in spinal fluid taken from AD patients. Although the function of tau, in the initiation of AD is not obvious, a parallel can be drawn with the behaviour of other microtubule proteins in epithelial cells which respond to transduction of membrane receptors by an unusual ligand. It has been shown that the interaction of enteropathogenic E.Coli (EPEC) within the membrane of cultured epithelial cells results in cytoskeleton rearrangement and hyperphosphorylation of three cytoskeletal proteins which migrate to a point below the site of membrane ligand interaction. The abnormally phosphorylated proteins form rigid aggregates (Rosenhine Llan, EMBO. J. 11 : 3551-3560 (1992)) which are reminiscent of aggregates (NFTs) formed by tau in AD brains.

Mutations (several) in the APP gene segregate with an autosomal dominant, early onset form of AD in a few families (Goate, A. M. et al, Nature 349:704-706 1991; Selko, D. Scientific American 265:40-47 1991; Hardy, J. et al. WIPO WO 92/13069 1992) mutations in S182, segregate with familial AD [FAD] (St. George-Hyslop, P, et al. Nature Genet. 2: 330-334 (1992); Sherrington, R. et al. Nature 375:754-760 (1995)); a mutation in STM2 segregates with a rare autosomal form of AD (Levy-Lahad, E. et al. Science 269:973-977 1995), and inheritance of the ApoE4 allele appears to be a susceptibility factor in all the above mentioned forms of AD (Corder, E. H. et al. Science 261:921-924 1993), while inheritance of the ApoE2 allele appears to confer a decreased risk (Corder H. L. et al. Nature Genet.7:180-184 1994).

Nevertheless, the combined genetic factors account for only a small percentage of AD and, in spite of the identification of the latter factors, little is known or can be inferred about the biochemical cause of the disease symptoms.. About 80 % of all AD which occurs mainly in humans > 80 years old does not appear to be associated with genetic predisposition.

The gene predisposing to susceptibility to AD.

### The gene products of genes predisposing to AD and DS

### I. The APP gene on chromosome 21

The APP gene is preferentially expressed in the neuronal cells of the central nervous system. The gene encodes a trans-membrane protein which contains a single monomeric trans membrane alpha helix (Kang J. et al. Nature 325:733-736 (1987); Tanzi, R.E. et al. Nature 321:528-530 1988)). The protein, which is expressed primarily in brain, can exist as several isoforms of various length caused by alternative splicing or by incorporating or shedding exons (Pollwein, P. et al. (Nucl. Acids Res. 20:63-68 (1992); Price, D.L. et al., Clin. Neuropharm. 14:S9-S14 (1991)), a feature it appears to have in common with the LDL receptor protein. However, all the isoforms have a common domain called the β Amyloid domain (Aβ), that is partly embedded within the membrane spanning region of the APP protein. The major product of post translational cleavage of APP protein is Aβ (Podlisny, M.B. et al., Science 238:669-671 (1987); Currie, J.R. et al., J. Neurosci. Res. 30:687-689 (1991)). (Zain, S.B. et al., Proc. Natl. Acad. Sci. (U.S.A.) 85:929-933 (1988); Vitek, M.P. et al., Molec. Brain Res. 4:121-131 (1988); Johnson, S.A. (In: Review of Biological Research in Aging, Vol. 4., Rothstein, M. (Ed.), Wiley-Liss, NY, 163-170 (1990)). The secretion of the Aβ protein thus reflects the cleavage of the domain from the precursor molecule (see, Roch, J.M. et al., J. Biol. Chem. 267:2214-2221 (1992)).

AS indicated above the pathological hallmark of AD, and DS, is the presence of intracellular tangles, and extracellular deposits or "plaques" of amyloid protein Aβ in the neuropil and in blood vessels (Mann, D.M.A. Neurobiol Aging 10:397-399 (1989); Selkoe, D.J. Neuron 6:487-498 (1991); Lampe, T.H. et al. Ann. Neurol. 36:368-378 (1994); Selkoe, D.J. Nature 375:734-735 (1995);). The principal component of the amyloid protein plaques in normal humans is Aβ, a 38-40 amino acid (~4kDa) hydrophobic protein (Price, D.L. et al., Clin. Neuropharm. 14:S9-S14 (1991)). Aβ forms the core of fibrils, which are concentrated in amyloid deposits in the extracellular space of the brain parenchyma and in the vascular elements of the brain and the pia-arachnoid (Currie, J.R. et al., J. Neurosci. Res. 30:687-689 (1991)). However, in Alzheimer's disease a longer version of Aβ, Aβ-42 is released from APP and it is this species of Aβ which is believed to be the major component of amyloid deposits and was also believed to be the neurotropic factor in AD (Roher A.E. et al.Proc Nat Acad Sci USA 90-10836-10840 (1993); Games, D. et al Nature 373:523-527 (1995); Laferia, F.M. et al., Nature Genet 9:21-30 (1995)), and in DS (Iwatsubo, T. et al., Ann. Neurol. 37:294-299 1995; Neuron 13:45-53 1994). Recently, it has been demonstrated in mice that the presence of a functional APP gene is not necessary for the formation of AD type neuropathology (Zengh, H. , et al. Cell 81:525-531 1995), also that synaptic loss can occur by overexpressing APP without plaque formation (Mucke, L. et al. Brain Res. 666:151-167 (1994)).

Some of the above mentioned experimental findings appear to argue against a role for Aβ in the neuropathology of AD; nevertheless, the early build up of Aβ in all forms of AD, (Querfurth, H. W. et al. Molec. Brain Res. 28:319-337 (1995); Levy-Lahad E., et al. Science 269:973-977 1995)), and the mutations in the APP gene which segregate with the disease, suggest a central role for APP in AD (Hardy, John. Proc.Natl. Acad. Sci 94:2095-2097 (1997)).

The accumulation of Aβ-42 in Alzheimer's disease is believed to result from the faulty processing of one or more of the APP isoforms (Currie, J.R. et al., J. Neurosci. Res. 30:687-689 (1991)), (see also, Johnson, S.A. In: Review of Biological Research in Aging, Vol. 4., Rothstein, M. (Ed.), Wiley-Liss, NY, 163-170 (1990); Roch, J.M. et al., J. Biol. Chem. 267:2214-2221 (1992)). Such processing is thought to involve two or more specific proteases (Azuma, T. et al., J. Biol. Chem. 267:1609-1613 (1992); Nakanishi N. et al., Exp Neurol 121:125- (1993)). At the moment there is a concerted effort by researchers to find these proteases, ("secretases") which essentially remain hypotethical to date; the method of Aβ overproduction in AD remains unknown.

### II. Apolipoprotein E located on chromosome 19

The E4 allele of apolipoprotein, "ApoE4", originally associated with late onset familial and sporadic Alzheimer's disease (Saunders, A.M. et al., Neurology 43:1467-1472 (1993); Namba, Y. et al., Brain Res. 514:163-166 (1991)) appears to be associated with all forms of inherited HD and DS. The gene is widely expressed but the highest expression is found in brain. ApoE4 is a member of a family of genes that also includes ApoE2 and ApoE3 (Utermann, G. et al., J. Lipid Res. 25:378-382 (1984); Utermann, G., J. Inher. Metab. Dis. 11:74-86 (1988); Ghiselli, G., et al., Lancet 2:405-407 (1982)). This gene family has been implicated in other serious human diseases involving faulty lipid metabolism, e.g., cardiovascular diseases. ApoE proteins bind to specific sites on the LDL receptor and on the related LRD receptor on cell membranes and serve as anchor sites for particular lipoproteins which are then transferred across the plasma membrane in coated pits and it has been shown that APP can bind to ApoE4 LDL receptors (Kounnas, M. Z. et al. Cell 82:331-340 1995) and hence may share a similar metabolic pathway; however, this gives little insight into how ApoE4 influences susceptibility to AD. ApoE proteins, like all soluble membrane proteins, aggregate in solution and ApoE4 is associated with Aβ immunoreactivity in β amyloid plaques. The latter might suggest that intra molecular protein aggregation could play a role in the pathogenesis of AD. The finding that ApoE2 which has a cys residue at position 112. whereas ApoE4 does not, confers a decreased risk to AD to people having both alleles, suggest that a cys amino acid residue may confer the ApoE2 effect, and suggest that a cys residue may be involved in aggregation processes relevant to AD (Shi Du Yan et al., Nature 382:685-691 (1997); (Tabatin, M.et. al., Neurobiology of aging 17 (48) 5130 (1997))

### III. S182 located on chromosome #14

Although the number of humans genetically predisposed to AD is relatively small, S182 is considered to be the major AD locus because the vast majority of Familial AD (FAD) patients have mutations in this region. The S182 gene encodes a transmembrane (TM) protein "AD3" which contains seven transmembrane helices. Five point mutations which occur in amino acid residues located in, or close to, different TM-helices, segregate with AD. Since the mutations were not found in normal humans they appear to be pathogenic for the AD phenotype in humans. The function of AD3 is unknown, but possible functional homology with SPE-4, a transmembrane protein expressed in the worm *Caenorhabditis elegans,* might suggest that AD3 can function in the cytoplasmic partitioning of proteins (Sherrington, R. et al. Nature 375:754-760 (1995); Hardy, John. Proc.Natl. Acad. Sci 94:2095-2097 (1997)).

### IV. STM2 located on chromosome #1

Like S182, the STM2 gene encodes a TM protein that contains seven transmembrane helices. The STM2 protein is highly homologous to S182, especially to sequences within the TM helices. A single point mutation in amino acid (aa) 141 located in the region of the second TM helix, which is 84 % homologous to AD3 TM-II, was found to be the cause of AD in a large kindred family. As compared to FAD caused by mutations in S182, FAD caused by the STM2 141 mutation appears to be confined to one kindred, "The German Volga family". The structural similarity between AD3 and STM2 protein indicates that they share a similar biochemical function which is presently unknown (Levy-Lahad, E. et al. Science 269:973-977 1995). However the secondary structure of S182 and STM2 closely resembles that of the adrenergic and muscarinic receptors. In the latter, several transmembrane domains are required for determining selectivity of antagonist and agonist binding (Wess, J. et al Mol. Pharmacol. 256:872-877 (1991)). This might explain why any one of the 5 mutations which occur in S182 can lead to AD.

Without some type of effective treatment, AD will probably affect about one out of every 10 humans alive today.

There is no effective treatment for AD at any stage of its clinical progression and no reliable diagnostic method to detect the disease at early stages, even within the relatively small number of people genetically predisposed to the disease.

In view of the importance of diagnosing, predicting, and treating AD and DS, affective means for achieving these goals are pressing. The present invention supplies such means.

### SUMMARY OF THE INVENTION

The object of the present invention was to provide highly specific molecules in humans which were highly reliable markers of AD in humans. These molecules, in addition to being accurate predictive agents for the diseases, were also targets for in situ modification or destruction by therapeutic substances which would prevent and stop the progress of the diseases in humans without undesirable side effects.

According to the invention the highly specific molecules which are markers for AD and also targets for therapeutic intervention have been provided.

The invention concerns agents and use of these agents as markers for early diagnosis and targets for therapeutic approaches for Alzheimer's disease. Such agents include three novel neuropeptides implicated in Alzheimer's disease and one novel neuropeptide implicated in Down syndrome (trisomy 21) as well as analogues and derivatives of these molecules, and nucleic acid molecules encoding such molecules, or influencing their expression.

### BRIEF DESCRIPTION OF THE FIGURES:

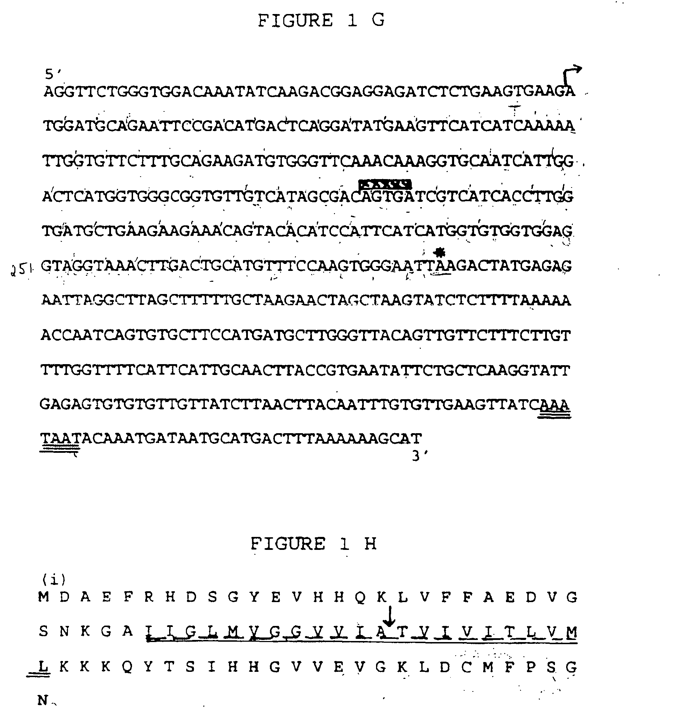

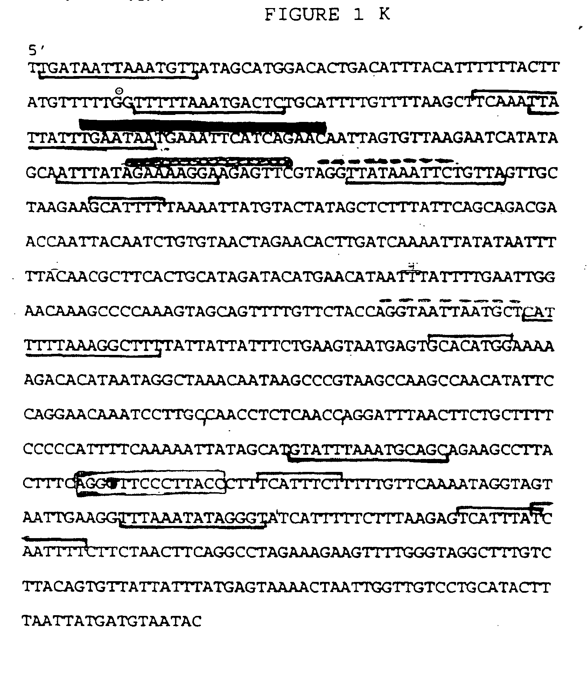

Figure 1G. Nucleotide sequence of alzas.
Figure 1H.
   (i) Amino acid sequence of ALZASp
   (ii) Amino acid sequence of ALZASp3
   (iii) Amino acid sequence of ALZASp4
Figure 1I. Nucleotide sequence of alzas1 cDNA
Figure 1J. Amino acid sequence of ALZASp1
Figure 1K. Nucleotide sequence of the 5' upstream regulatory region of alzas gene.
Figure 1L. Nucleotide sequence of alzas2 cDNA.
Figure 1M.
   (i) Amino acid sequence of ALZASp2
   (ii) Amino acid sequence of ALZASp5
Figure 1N. Nucleotide sequence of the 5' regulatory region of alzas2 gene.
Figure 2A. Organisation of alzas gene, comparison with organization of APP gene: products expected from constitutive cleavage of ALZASp and from mutations in regions in chromosome# 21 encoding APP.
Figure 2B. Organisation of alzas1 gene, comparison with organization of APP gene: products expected from constitutive cleavage of ALZASp1 and from mutations in regions in chromosome# 21 encoding APP.
Figure 2C. Organization of alzas2 gene, comparison with organization of APP gene: products expected from constitutive cleavage of ALZASp2 and from mutations in regions in chromosome #21 encoding APP gene.
Figure 3 A, 3B & and 3C. A Expression of AD related mRNA in human brain and lymphocyte: (A) amplification with primer pair "alz289 - lane 1= DS brain, lane 2 & 3= normal brain, lane 4 = hippocampus, lane 5= AD cortex, (only a small amount of the PCR product was applied to the gel) lane 6 & 7= DNA size markers, lane 7= normal lymphocyte, lane 7= AD lymphocyte; amplification with primer pair "alz188"- lane 1 & 4= DNA size markers, lane 2= AD brain, lane 3 = normal brain; 5 & 6 = normal lymphocytes, 7 & 11-14 = normal brain 15 = DNA size markers.
Figure 4 A-B. ELISA methods
   A. Description of the ELISA method used to detect ALZAS and DSAS and PAC in human body fluids and tissue.
   B. Description of the ELISA method used to detect endogenous immunoglobulins produced in blood and serum of patients with A0.
   C-E Detection of expression of AD related proteins ALZASp (ALZAS), detection by immuno-chemiluminescent detection on dot blots.
      For dot blots, proteins extracted from brain and lymphocyte by ammonium sulphate precipitation were dialysed and boiled for 5 minutes in 5% SDS. 2µl of the SDS treated proteins were spotted onto nylon membranes, treated with the appropriate antibody and detected with an immuno-chemiluminescent system: (A) probe = antibody ALZab2.
      (C) dots were probed with antibody ALZab1; dots 1= normal lymphocytes, 3= DS lymphocytes, 6 = normal brain, 7 - = AD cortex, 8 & 9 - = AD lymphocytes, 10 = normal fibroblast;
      (D) 1.5 µl serum obtained from ten normal women over 35 years old selected at random was probed with ALZab1.
      (F) Dot blots of serum from 28 autopsy confirmed AD victims was probed with ALZab2 (at least 50% of these patients we incorrectly diagnosed for a condition other than AD before death);
      (G) Detecting different levels of ALZAS in serum from suspected early AD patients, patients with depression and autopsy confirmed AD victims.
      (H) Detection of endogenous anti-ALZAS IgG in patients blood, serum and saliva
      (I) western blots; 1 proteins in ammonium sulphate precipitates were electrophoresed on 17.5 % acrylamide gels, blotted onto nylon filters, reacted with the appropriate antibody and detected with the immuno-chemiluminescent system, 2. proteins were isolated from AD hippocampus and treated with antibody ALZab2; 3. proteins were isolated from AD hippocampus and created with ALZab3;
Figure 5A-D
   (A) Isolation of ALZAS from serum obtained from a single autopsy confirmed AD patient. The protein was isolated using affinity purification methods on affinity purified anti ALZab2 antibody linked to CNBR-sepharose. The gels were stained with silver stains and western blotted. Human IG fragments were detected with mouse anti-human mABs.
   (B) Silver stain of SDS gel: proteins obtained following affinity purification of ALZAS from serum from a late stage AD patient.
   (C) Detection of ALZAS-IgG complexes present on gel of 5B with anti-human IgG (Fc fragment).
   (D) Western blot of cationic non SDS polyacrylamide gel following electrophoresis of serum from (a) a patient with sporadic AD, and (b) a patient with swedish mutation AD.
Fiqure 6 Alignments:(A)alignment of S182 tm-II.With ALZASpTM (APP-TM) and STM2 TM-2.
   (B) alignment of c-terminal of ApoE4 with the c-terminal of ALZASp1
   (C) aligment of the receptor/heparin binding site of ApoE proteins with the c-terminal ALZASp1.

### DETAILS OF THE INVENTION

In detail, the invention provides a nucleic acid molecule substantially free of natural contaminants, that encodes a protein selected from the group consisting of alzas, alzas1 and alzas2. In particular, the invention provides the above-described nucleotide acid molecule wherein the sequence is SEQ ID NO:11.

The invention also provides a protein, sbstantly free of natural contaminants, selected from the group consisting of ALZASp, ALZASp1, ALZASp2. In particular, the invention provides the above-described protein having a sequence of SEQ ID NO:12. The invention also provides three associated hypothetical proteins having a sequence of SEQ ID NO:13, SEQ ID NO:14 and SEQ I(D NO:26.

The invention also provides a reagent capable of diagnosing the presence of a molecule selected from the group consisting alzas, a ALZAS-encoding nucleic acid molecule, alzas1, a ALZAS1-encoding molecule, alzas2, a ALZAS2-encoding molecule.

The invention particularly concerns the embodiments wherein the reagent is a nucleic acid molecule produced from nucleic acid molecules having a sequence complementary to SEQ ID:NO 11 (nucleotides 191-240), or SEQ ID:NO 24 (nucleotides 96-170), or a nucleic acid molecule obtainable by mutating a nucleic acid molecule having a sequence of :SEQ ID:NO 11 (nucleotides 191-240) SEQ ID:NO .24 (nucleotides 96-170), or a protein (especially an antibody, or a fragment of an antibody, which is capable of binding to ALZASp (amino acids 68-79), ALZASp1 (amino acids

The invention also provides a method of treating Alzheimer's disease, in need of such treatment, an effective amount of an antibody or anti peptide substance against ALZASp, or of a reagent to block the activation of the promoters and other regulatory elements which program transcription of alzas, or the heat shock elements which can influence activity of such promoters within the sequences identified as, SEQ ID NO:17, SEQ ID NO:18; SEQ ID NO:19, SEQ ID NO:20, SEQ ID NO:21, SEQ ID NO:22, SEQ ID NO:23, SEQ ID NO:27, SEQ ID:NO 22
5' TAGCATGTATTTAAATGCAGCAGAAG 3'

### DESCRIPTION OF THE PREFERRED EMBODIMENTS:

The consensus of all experimental evidence suggests: (1) that APP protein (the β amyloid precursor) plays a major role in the expression of AD disease phenotype regardless of origin of the initial biochemical trigger of the disease; the involvement of APP is manifested by the abnormal deposits of a variety of Aβ molecules in crucial regions of the brain, (2) Aβ is a 38-42, 4.6kd polypeptide which is derived from post-translational cellular processing of APP protein, i.e. it is a fragment of the β amyloid precursor protein (see Selkoe, D.J. Neuron 6:487-498 (1991), Nature 375:734-735 (1995); Masters, C. L. et al. Proc. Natl. Acad. Sci. USA. 82:4245-4249 1985)) (3) the presence of Aβ in brain and cerebral arteries signals the beginning of AD, although Aβ does not cause the neurodegenerative symptoms characteristic of AD; (4) the APP gene is not overexpressed in AD although it is overexpressed in Down Syndrome because of the additional gene dosage, but the same might apply to other chromosome 21 associated genes that have no involvement in DS, (5) the emphasis for finding the biochemical cause of AD has shifted to finding the proteases responsible for faulty cellular processing of APP and, hopefully, targeting these for diagnostic and therapeutic, finally (6) in the meantime, there is neither a reliable non invasive method to detect AD in the early stages nor an effective method to treat the disease.

The success of the present invention came mainly from our realization that there must be other genes within the APP locus with association to AD and, and that at least one of these genes must also have a β-amyloid related component. Therefore, we used a procedure which we had successfully used to find alternative genes, which are putative causative factors of other "genetic diseases", to search for such genes which might segregate with AD, within the locus encoding the entire APP gene on chromosome 21 and the regions that flank the gene.

We call these alternative genes "piggy-back genes", and we refer to this technology as "disease gene discovery by positional searching" (DGDPS). Piggy genes are transcribed in any orientation within the chromosomal locus occupied by another gene.

### DGDPS procedure.

This procedure has an advantage over gene isolation by cloning from a genomic or cDNA library, because it overcomes three important drawbacks, (1) the possibility that some DNA sequences cannot be cloned by the conventional methods, (2) that some mRNA sequences are of such low abundance that they are not represented in the cDNA library, and (3) the products of some cloned sequences are highly toxic to bacterial or other hosts.

In general, first we identified a gene closely related to a gene already genetically linked to a certain disease, then isolated the mRNA transcribed from the gene from disease tissue or patient's blood, then synthesized cDNA from the isolated mRNA with reverse transcriptase then amplified the novel cDNA with specific primers which flanked the entire coding region of the cDNA, then we identified the cDNA from the size following electrophoresis on agarose gel, and finally isolated the unique cDNA from the agarose gel. This allowed us to select out the desired molecule, if it was expressed, without having to probe several million cDNA clones.

The fact that Aβ, which is present in all AD, is a part of the APP protein, and other indications from results of our work with some other neurodegenerative diseases, we concluded that the biochemical link between all forms of AD was the APP protein. We hypothesized that Aβ was not directly generated from APP embedded in the membrane, rather it came either from "soluble" i.e., transported APP or from a different protein which was likely to be expressed in significant amounts in a disease specific manner. We called the protein "ALZAS" (Alzheimer's disease associated), and we predicted that ALZAS would be structurally closely related to APP and transcribed from within the same chromosomal location as APP. Furthermore we predicted that ALZAS would influence the phosphorylation of tau, would initiate the immunological reactions which lead to complement formation in neurons and ALSASp would be present in body fluids (serum, saliva, urine) of people with AD. The aim of this project was to find ALZAS in patients with AD.

The present invention derives in part from the discovery and cloning of a novel gene alzas, using the above approach. Following is a detailed description of positional searching as it applied to the present invention:
(1) We examined the sequenced regions within the APP locus on chromosome 21 and selected potential complete orf's, i.e. with acceptable translation initiation sequences (see Kozak, M. Nucleic Acid Res. 12:857-872 1984) and translation termination stop codons (TAA, TAG or TGA) in place,
(2) next we used the method of Bucher et al., J. Mol. Biol. 212; 563 - 578 (1990) to identify putative promoter regions associated with the orf within 100-1000 bp 5' upstream of the translation initiation sequence and we identified potential poly-A addition signals (the consensus poly-A addition sequence is AATAAA) within a region of -1000 bp 3' downstream from the stop translation codon of the potential orf,
(3) then, orf's fulfilling the above two characteristics were translated into putative protein sequences using the universal code,
(4) then we analyzed the putative protein with our proprietary computer assisted protein finger printing technology and obtained information about the potential biochemical characteristics of the deduced proteins,
(5) next the biochemical characteristics of the deduced proteins were correlated with known clinical symptoms of the AD (Mann, D.M.A Neurobiol Ageing 10:397-399 1989), and with the biochemical characteristics of the disease reported (Selkoe, D.J. Neuron.6:487-498 1991),
(6) RNA encoding proteins with properties correlating with the disease characteristics were selected as potential disease related candidates,
(7) detection of the presence of transcribed mRNA sequences encoding the protein in a cell was done by PCR, (Mullis, K.B., Cold Spring Harbour Symp. Quant. Biol. 51:263-273 (1986); Saiki, R.K., et al., Bio/Technology 3:1008-1012 (1985); Mullis K. et al., U.S. Patent 4,683,202; Erlich, H., U.S. Patent 4,582,788; Saiki, R. et al., US 4,683,194 and Mullis, K:B., et al. , Met. Enzymol. 155:335-350 (1987); in detail reverse transcriptase-PCR (RT-PCR) was done using the Stratagene RAP-PCR RT-PCR kit according to the manufacturer's instructions, with unlabelled primers to detect cDNAs encoding the deduced proteins in RNA isolated from frozen human brain and lymphocytes.
   RNA from frozen brains were extracted by grinding postmortem frozen brains in a tissue homogenizer in the presence of diethylpyrocarbonate (DEPC). Total RNA was isolated using the Stratagene micro RNA isolating Kit and poly (A)+ was isolated using Stratagene Poly(A)+ Quick mRNA isolation kit following the manufacturer's instruction. Isolated RNA was treated with 10 units of RNAse free DNAse at 37oC for 15 minutes. DNAse was inactivated by treating for 2 mins at 100 oC. cDNA synthesis using mRNA as template was carried out with the first strand protocol supplied with the and RT-PCR was done using the cycling conditions recommended by the manufacturer. Forward and reverse PCR primers were prepared to regions flanking the entire protein coding region of the orf of the selected protein (see table 1 for sequence of the primers and for the size of the expected amplified product). The amplified cDNA was electrophoresed on agarose gels and the size was determined by comparison with DNA size markers which were electrophoresed alongside. To verify the sequence of the cDNA, the region of agarose containing the desired size cDNA was extracted into H2O, precipitated with ethanol and a portion was cycle sequenced using the primers in "12" and Perkin Elmer ampli-Taq on the Perkin Elmer 376 A DNA sequencer using a non radioactive method described by Liu, C. et al.Nucl. Acid. Res 21:333-334 (1993).
(8) To determine if the proteins were actually expressed, epitopes were identified within the amino acid sequence of the protein using the method of Hopp, T.P. and Woods, K.R. Proc. Natl Acad Sci. USA 78:3824-3828 (1981) and their sequences compared to sequences in databases; epitopes (see table #2) having no homologue within the public databases were selected and monospecific polyclonal rabbit antibodies were prepared against these and purified by immunoaffinity chromatography on Pharmacia LKB, CNBr-activated sepharose 4B according to the recommendation of the manufacturer (see section #11 on Immunology, in Current Protocols in Molecular Biology (Vol 1) Ausubel, F.M. et al (ed) John Wiley & Sons NY. NY. 1991).
9. To detect the expression of the deduced proteins in human material, proteins were isolated from AD, and normal tissue, by precipitating the homogenized tissue with >80% ammonium sulphate and dialysis against SDSPAGE buffer (the buffer conditions are described in Laemmeli, U.K. Nature 227:680-685 (1970)), (16) dialysed proteins were boiled in SDSPAGE buffer and either electrophoresed in 17.5% acrylamide gel, following which the proteins were Western blotted onto nylon membrane and treated with the affinity purified antibody or spotted onto positively charged membranes and treated as above. Interaction of the antibody with the protein bound to the membrane was visualized with a chemiluminescent kit purchased from BioRad Inc according to the manufacturer's instructions (also see Blake M.S. et al.Anal. biochem. 136:175-179 (1984).

ALZAS has structural similarities to small pore-forming proteins. Pore-forming proteins found in a variety of organisms penetrate cellular membranes and mediate membrane damage, usually at a very low effector/target cell ratio. For a pore forming protein to be effective, two interconvertible configurations of the protein must exist in the cell: (i) for membrane insertion the protein must present a hydrophobic domain on the surface which must not be dominant, (ii) whereas for traversing the cytoplasm to reach the membrane the protein must behave as a soluble entity. The interconvertible configurations might be achieved in several ways including oligomerization, or interaction through hydrophobic sites with a chaperon. In order to enter the membrane, on contact with the membrane dissociation of the oligomer occurs which re-exposes the protein hydrophobic domain and drives the membrane insertion. ALZAS has the APP transmembrane signal; therefore, like APP, ALZAS can be translocated across the membrane of the endoplasmic reticulum (ER) and the cytoplasm and can insert into the same sites as APP in plasma membranes.

The gene alzas, and the protein encoded by this gene are described in the examples given below. Our discovery that these molecules: (i) were expressed in (100%) of brains, lymphocytes and blood obtained from humans with AD, (ii) elicited an antibody response in humans before clinical symptoms of the disease was detected, (iii) were not detected in normal individuals (normal = individuals below 30 years and individuals above 60 with no history of a neurodegenerative disease), and (iv) in accordance with the known incidence of AD in the population, were detected in 2 of 5 clinically normal people over the age of 65 who appear suspect for AD. Strongly supported involvement in the etiology of AD and DS. Furthermore it was a strong indication that these molecules could be used for presymptomatic diagnosis of AD, and furthermore were targets for active and/or passive vaccines type therapeutics to prevent and STOP AD. THE MOLECULES OF THE INVENTION

### Example 1

### Example 2

### The ALZAS transmembrane protein.

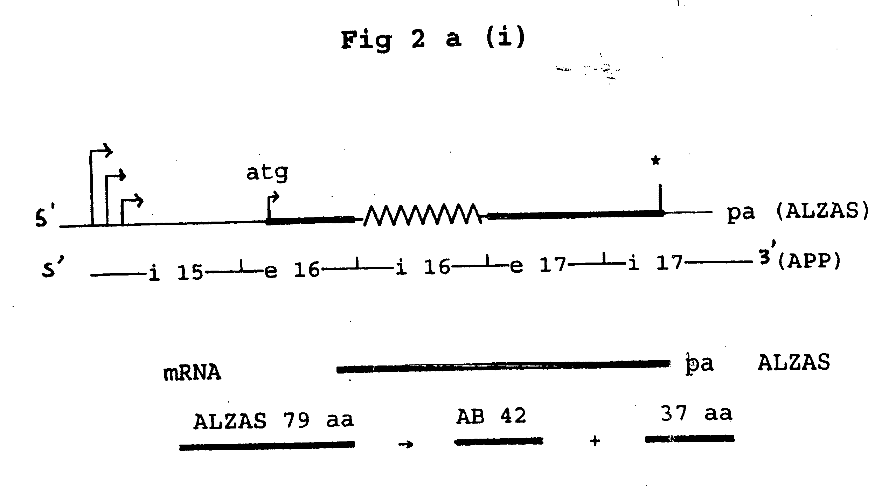

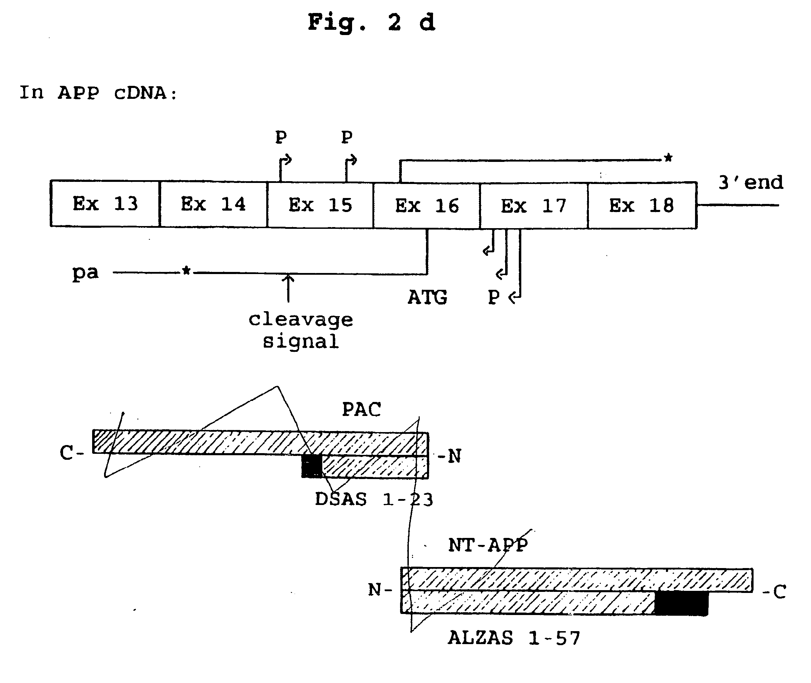

The invention also relates to a gene which we call Alzheimer's associated ("alzas"). alzas is encoded within the APP locus on chromosome 21. The positional relationship to APP encoding sequences is shown in figure 2Ai. The gene comprises two exons separated by a 5.6 kb intron. The nucleotide sequence of the transcription regulatory region of alzas, which lies within intron 15 of the APP gene, is shown in Figure 1 K. Transcription of alzas can be programmed by any of eight promoters, ("PALZ1")+("PALZ2") SEQ ID:NO 17; ("PALZ3") SEQ ID:NO 18, ("PALZ4") SEQ ID:NO 19, ("PALZ5") SEQ ID:NO 10, ("PALZ6") SEQ ID:NO 21, ("PALZ7") SEQ ID:NO 22 and ("PALZ8") SEQ ID:NO 23, located in the regulatory region. PALZ3/4/5/6/7/8 are correlated with cap sites. Heat shock elements, which can modulate activity of all the alzas promoters, overlaps PAL3 (Heat shock proteins are reviewed by Gething, M.J. and Sambrock, J. Nature 355:33-45 (1992); two putative estrogen responsive elements (Savouret et al., Recent Progress in Hormone Res. 45:69-120 1989; Beato M. Cell 56:355-361 1989), lie ahead of and between PALZ 7/8. A potential poly-A addition site is present in the 3' downstream untranslated region of the gene.

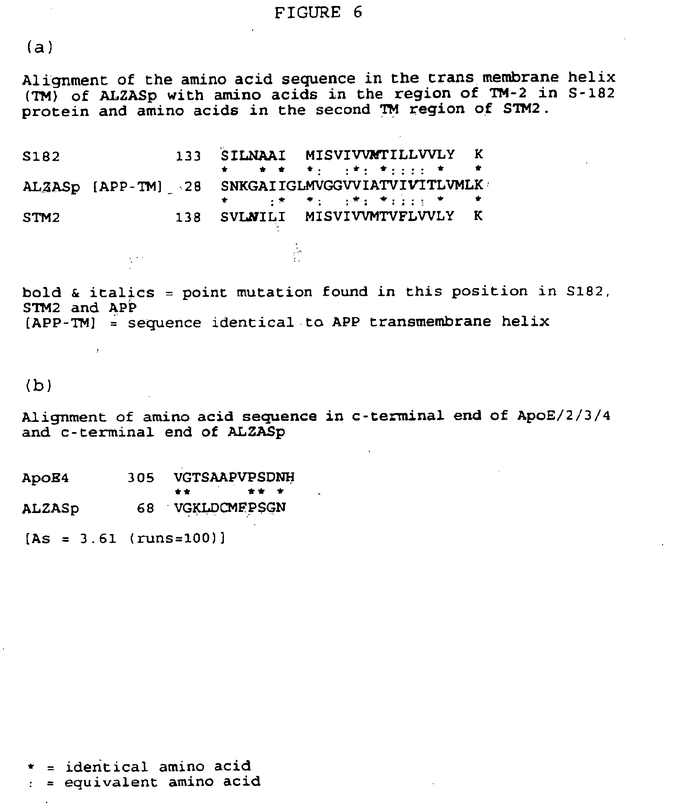

The alzas cDNA is shown in figure 1G SEQ ID:NO 11. It inclus sequences identical to APP exon 16, exon 17 and part of intian 17, and encodes a 79 amino acid protein ALZASp, SEQ ID:NO 12, shown in figure 1 Hi. The protein includes the complete Aβ protein sequence, the APP transmembrane helix sequence (which has a constitutive hormone controlled secretory signal between aa 42//43) and a unique c-terminal sequence that is not related to APP amino acid sequences but has significant homology to a domain in a plant chloroplast membrane protein, and to the c-terminal sequence of ApoE4 proteins (see figure 6b) as determined with the method described by Feng, D.F. et al. J. Mol. Evol. 21:112-125 (1985).

### How ALZASp might cause or initiate AD in humans.

The presence of a transmembrane helix (TM) identical to APP TM means that ALZASp (which is expected to have a far less complex secondary structure than APP because of size) can successfully compete with APP for membrane anchor sites and may prevent APP from inserting into membranes. When this happens APP protein becomes vulnerable to degrading lysosomal protease activity. The latter could generate increased levels and various sizes of Aβ protein fragments without any change in the actual level of APP may also inadvertently target at Aβ and the transmembrane sequence in APP protein. Joint proteolytic targeting of APP and ALZAS could explain all forms of AS and can furthermore explain how Aβ appears to, be cut in a portion of the APP which lies within the TM helix. This also obviates a functional significance for specific digestion of APP by unique proteases. Therefore, it appears likely that the condition of a human immune response might play a significant role as to who gets AD and it makes the role of inflammation in the etiology self explanatory. It is possible that disruption of cytoskeletal structure which leads to abnormal phosphorylation of tau by activated tyrosine kinases a direct result of ALZAS interaction with the neuron plasma membrane (See Knulton J. et al., Infect and Immuno., 57:1290-1298 (1989), Rosenshine et al., EMBO J. 11:3551-3560 1992)) in the same manner as production of multiple forms of Aβ is due to the exclusion of APP from the membrane by competitive ALZAS binding and subsequent digestion by proteolytic enzymes.

Given the above indications, it appears that DS and AD may not be as closely related as we think. The factor which connects the two diseases i.e., the presence of Aβ, which may have little physiological significance in AD and DS is likely caused by two opposite mechanisms each resulting in production of Aβ from free APP (i.e. associated with the membrane). In DS the accumulation of Aβ is due to the overproduction of APP, which over-saturates membrane sites and leads to exclusion of a significant amount of APP molecules, which are digested in the cytoplasm; in AD, APP is out-competed for membrane sites by ALZAS and remains in the cytoplasm where it is digested.

### Amplification of alzas in human frozen brains and lymphocytes

RNA was isolated from 13 frozen normal human brains and lymphocytes, frozen AD brains and lymphocytes. cDNA synthesis of mRNAs were done as described above and cDNA amplification was carried out with pp alz287 (see table la) (and with pp alz393 data not shown) which was expected to amplify a 287bp fragment.

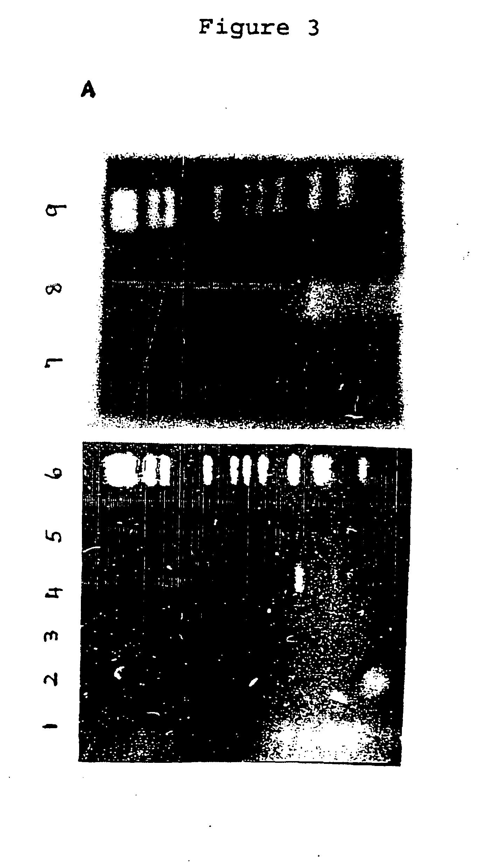

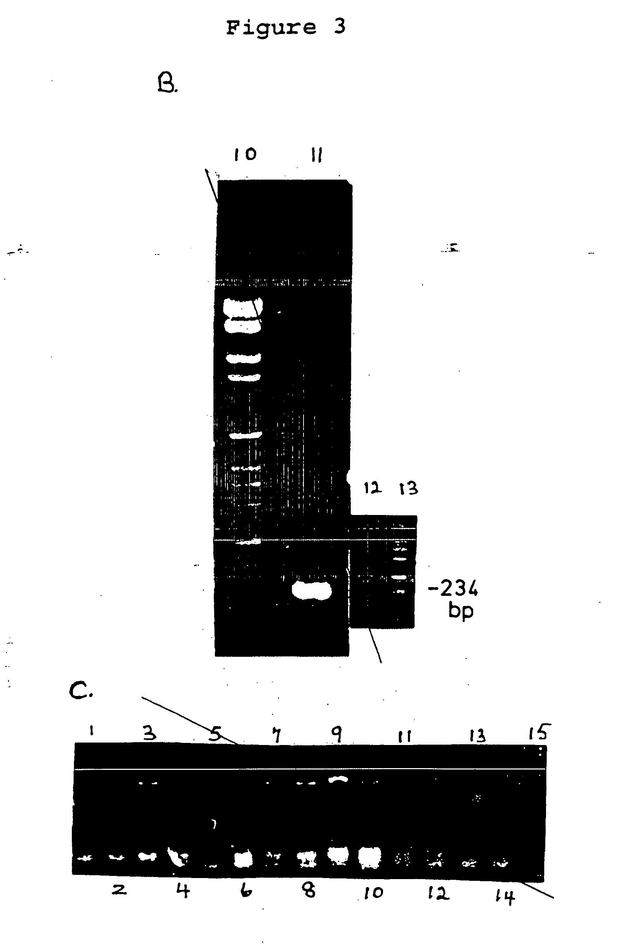

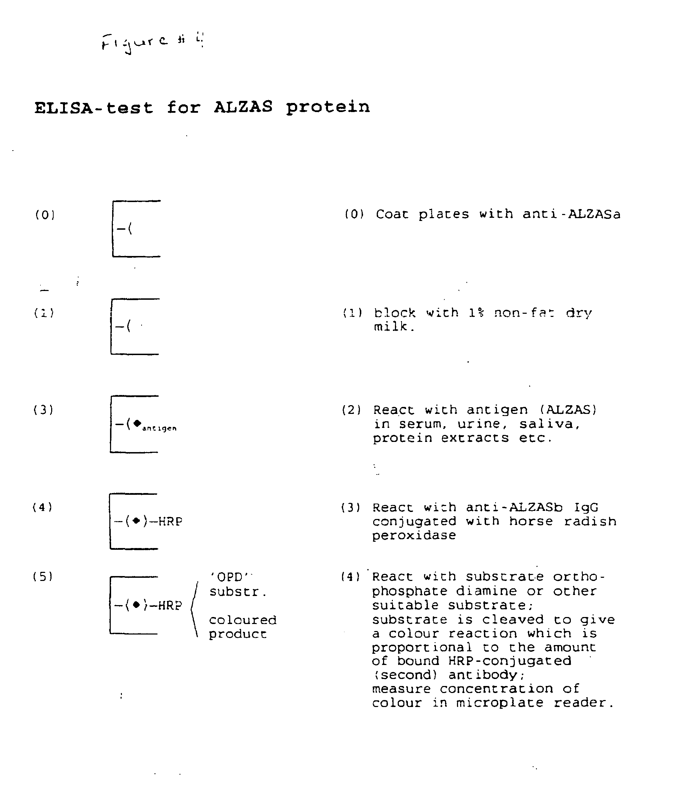

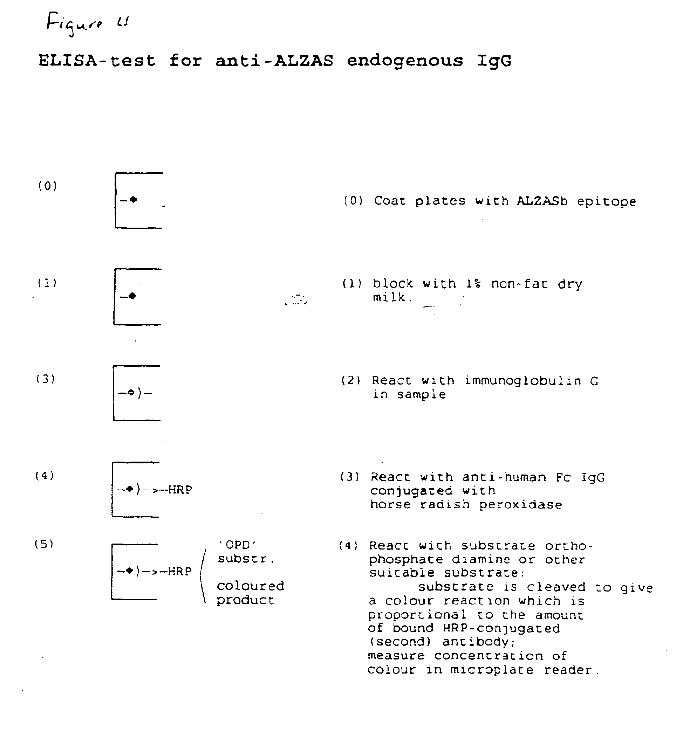

The amplified fragment was detected by comparison with DNA size markers electrophoresed under the same conditions (figure 3A). Lane DS brain, lane 2 & 3 normal brain, lane 4, AD hippocampus, lane 5, AD cortex, lane 6 & 9 DNA size markers (Boheringer, marker #5), lane 7, normal lymphocyte, lane 8 AD lymphocyte. The amplified 287 base pair fragment was isolated from the agarose gel and subjected to DNA cycle sequencing using the nonradioactive method described previously for pp 287. The results (not shown) matched exactly the predicted nucleotide sequence in figure IG.

### Detection of ALZASp in frozen human brains and lymphocytes

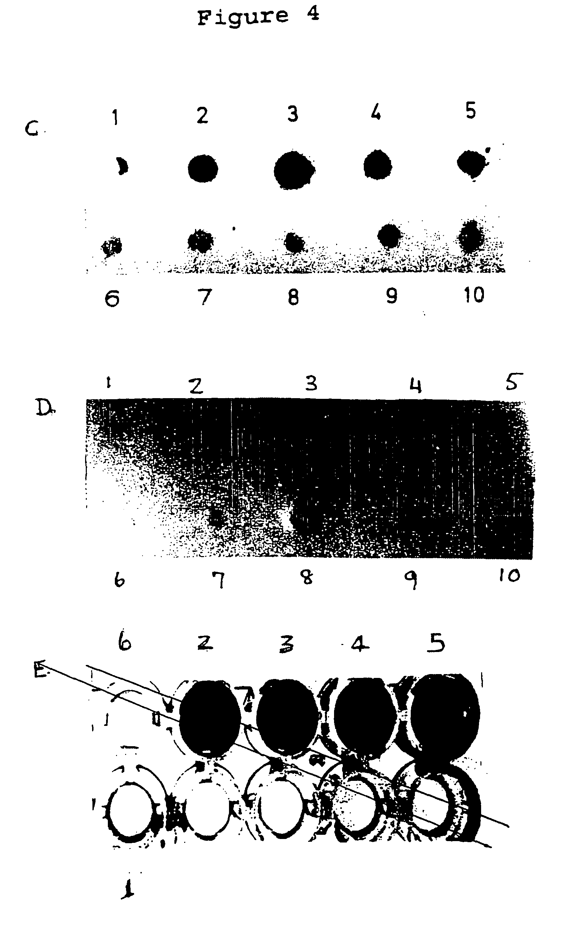

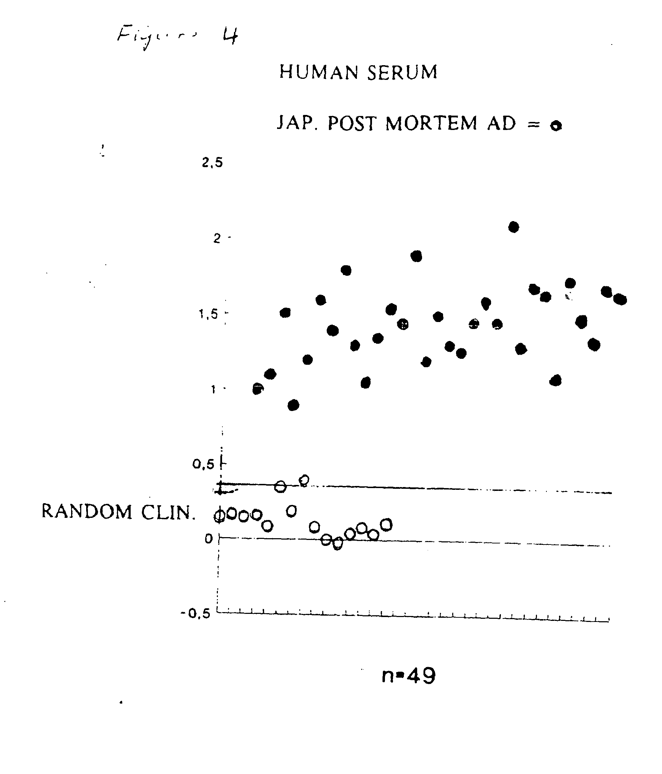

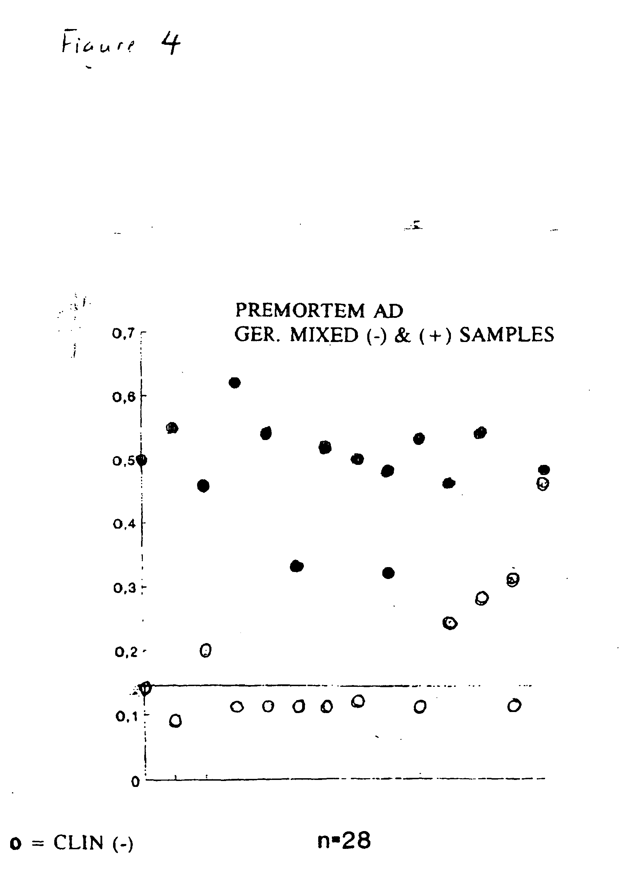

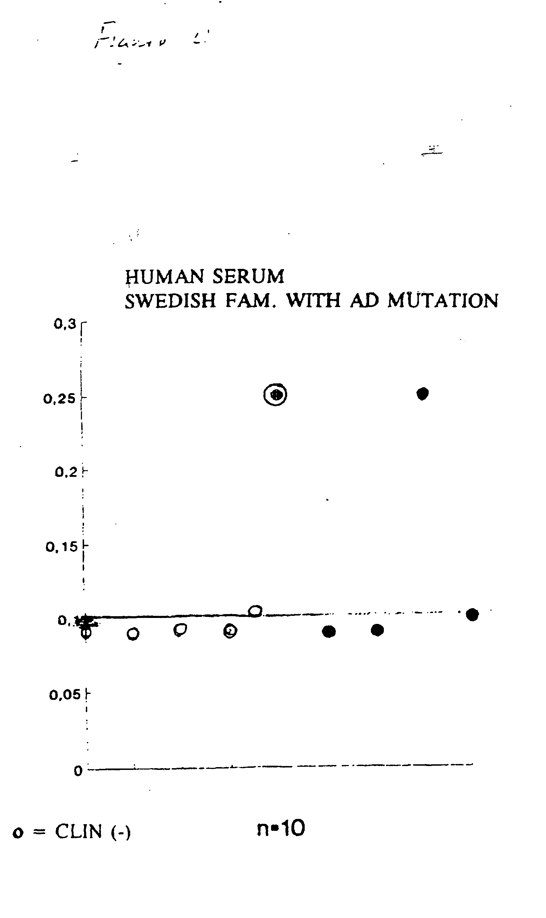

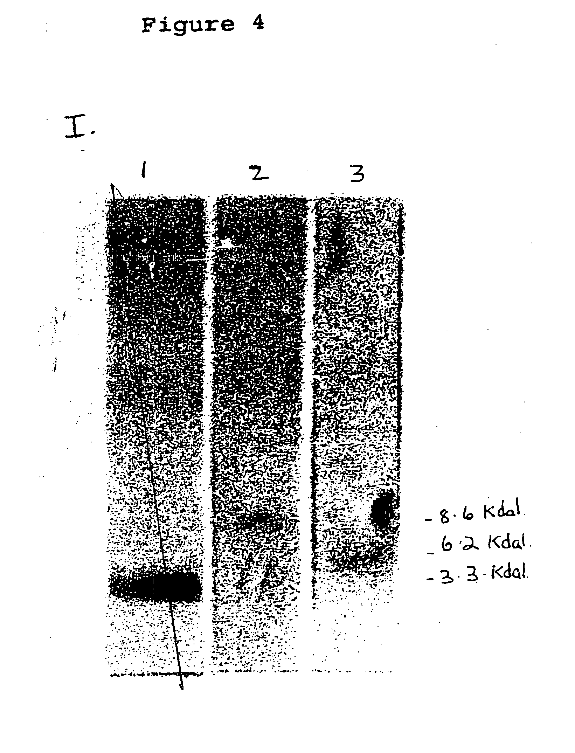

Aliquots of proteins isolated, as described above, from frozen human brain, frozen AD brain, normal lymphocytes and AD lymphocytes, were spotted on a nylon membrane or subjected to western blotting as described, and probed with antibody ALZab2.
ALZab2 detects only the last 12 amino acids in the c-terminal of ALZASp. The results in figure 4E &F show the presence of ALZASp in AD cortex and lymphocyte respectively.
Western blots, figure 4D, lane 2 probed with ALZab2 showed a single, immunoreactive, band of 8.5 kd which was the expected size of ALZASp.

The presence of multiple promoter elements, heat shock elements, and putative estrogen receptor elements in the regulatory region of alzas is an indication that transcription of alzas mRNA might be triggered by a number of different factors including external environmental factors unrelated to mutations, e.g., stress inducing factors, metals, etc. However, certain factors might have a suppressive effect on the activation of the promoters (e.g estrogens, nicotinic acid related molecules, estrogens) and hence may delay, or even prevent, AD in some humans. Therefore, according to this invention these promoters are ideal targets for therapeutics which will block activation and prevent transcription of alzas in humans.

### Example 1: Detecting ALZAS protein in serum from people with the

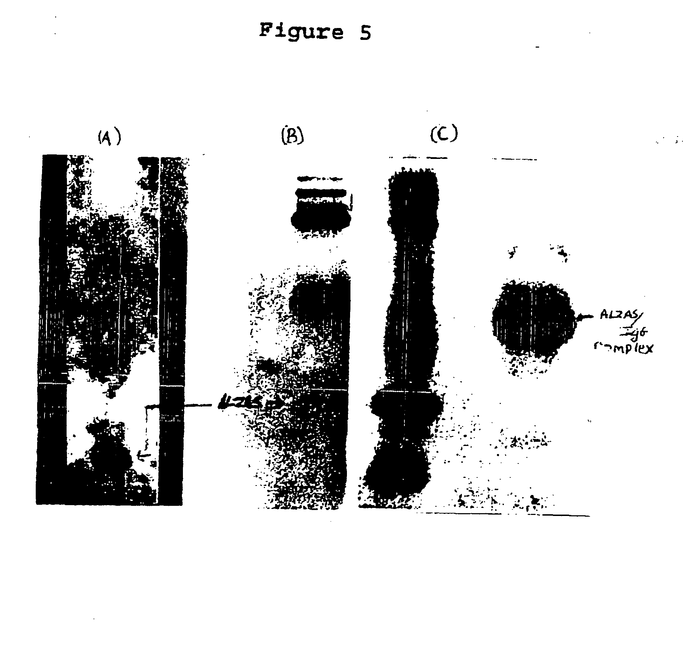

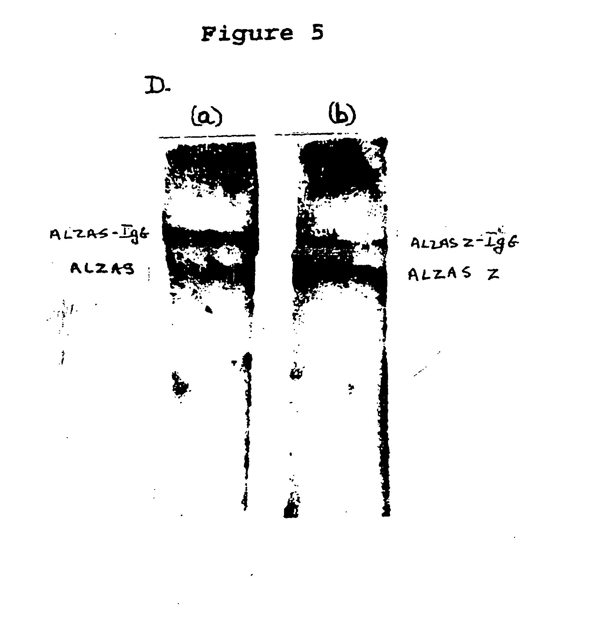

Swedish mutation using the ELISA procedure as in figure 4A. In figure 5 a, subjects 1-5 have no mutation, subject 6 has the mutation and has clinical AD, subjects 7-10 have the mutation but no clinical signs of AD. As can be seen, patients no 6 and no 9 had high levels of ALZAS.
In Example 2, figure 5 b, serum from a patient with sporadic AD, post mortem confirmed, and patient no. 6 from example 1, was subjected to cationic SDS-free polyacrylamide gel electrophoresis, western blotting and immuno-reacted with anti-ALZAS b. Positive reacting bands were visualized by subsequent treatment with chemiluminescence anti-rabbit IgG system. As can be seen, that ALZAS reacted with protein bands in both, the serum from sporadic AD and from the Swedish mutation AD. It can also be seen that the antibody interacted with a complex which we have found in other experiments, to be a complex of ALZAS and endogenous human anti-ALZAS IgG.

In summary, ALZAS2p when expressed can mimic some of the biochemical activities of ALSASp which is involved in AD.

### Effect of mutations in APP exons 16 & 17 on the expression of ALZAS proteins.

There is a remote possibility that the region of chromosome 21 where alzas, alsas1 and alzas2 are located may be a duplicate of a small region of chromosome 21. In this case, these genes may not be obligatory effected by mutations in the full length chromosome. However, it is more than likely that the genes are transcribed from within the APP gene, either in certain subsets of cells, or only when the promoters are sporadically activated by toxic intra/inter cellular factors produced in neuronal cells or toxic substances that enter such cells from the external environment. Other mutations in chromosome 21 which affect the configuration of DNA sequences in APP exons 16 and 17 and have been linked to early onset AD (Mullan, M. and Crawford, F. Trends neurosci . 16 : 398-403 1993), may occur also in the alzas genes. The Hardy VI and VG mutation, the VF mutation, and the Dutch EQ mutation that occurs in exon 17 (Selkoe, D.J. A Rev. Neurosci. 17:489-517 (1994); Hardy, J. Clin. Geriatr. Med. 10:239-247 1994)), cause a single amino acid change in ALZASp/p1/p2. However, the DM to QL mutation in exon 16 ( Cai, X.D. et al. Science 259:514-516 1993) eliminates the normal orf for ALZASp and ALZAS1p which can lead to translation and expression of alternative proteins using other reading frames. Two hypotethical neuropeptides ("[hyp]ALZASp"), shown in figure 1 Hii, and (" [hyp1]ALZASp"), shown in figure 1 Hiii, can be translated from alzas cDNA. Amino acid 23 -40 in [hyp1]ALZASp] is identical to the sequence of [hyp]ALZAS2p. If expressed, the hypotethical proteins might play a role in the etiology of AD, and also lead to variations of the disease phenotype e.g., the mutation at codon #713 that causes schizophrenia, Jones, C.T., et al. Nature Genet. 1: 306-309 (1992)), and the mutation at codon #702 that leads to hereditary cerebral haemorrhage, Dutch type (Levy, E. et al. Science:248 1124-1126 (1990)), may cause alterations in the biochemical property of at least one of the hypotethical proteins.
The following data driven deductions have been made concerniing AD

ALZASp has structural similarities to small pore-forming proteins. Pore-forming proteins found in a variety of organisms penetrate cellular membranes and mediate membrane damage, usually at a very low effector/target cell ratio. For a pore forming protein to be effective, two interconvertible configurations of the protein must exist in the cell: (i) for membrane insertion the protein must present a hydrophobic domain on the surface which must not be dominant, (ii) whereas for traversing the cytoplasm to reach the membrane the protein must behave as a soluble entity. The interconvertible configurations might be achieved in several ways including oligomerization, or interaction through hydrophobic sites with a chaperon. In order to enter the membrane, on contact with the membrane dissociation of the oligomer occurs which re-exposes the protein hydrophobic domain and drives the membrane insertion. ALZASp has the APP transmembrane signal; therefore, like APP, ALZAS can be translocated across the membrane of the endoplasmic reticulum (ER) and the cytoplasm and can insert into the same sites as APP in plasma membranes.

Internally, in response to adherence of certain infectious agents, or secretory products of such agents, to localized areas on cell membranes specific cytoskeleton proteins become highly phosphorylated by activated tyrosine phosphatases and form organised cytoskeleton structures. The latter interacts with the cell membrane directly below the point of contact on the surface membrane and this facilitate entry of the agent into the cytoplasm. Tau may be specifically mobilized and hyperphosphorylated in response to ALZAS interacting with membranes of specific sub sets of neurons. Externally, the entry of ALZAS into neuronal plasma membrane causes the release and binding of IgG, IgM and other immunological factors to the membrane bound protein, particularly the unique c-terminal sequence. This acts as a signal for the initiation of the complement cascade which targets cells bearing ALZAS for destruction and attempted removal.

In summary, the clinical symptoms of AD are initiated by a combination of ALZASp caused loss of APP from neuronal ER and plasma membranes and slow dissolution of the affected neuronal membranes by complement directed activity against ALZASp on the surface of the membrane. The latter leads to an autoimmune-like condition in those section of the brain affected by the disease.

**The Uses of the Molecules of the Present Invention.**

### A. Diagnostic Uses

ALZASp is expressed in all humans clinically positive for AD. ALZAS is also expressed in 2 out of 5 humans 65 years or older that are clinically normal for AD (some of which have other neurodegenerative diseases) whereas ALZAS has not been found in humans that are distinctly normal, i.e. humans <50 years old without any visible symptoms or history of the same. However, ALZAS has been found in saliva of menopausal women suffering from osteoporosis accompanied by depression. The detection of these molecules may be done by any of a variety of immunological methods (Yolken, R.H., Rev. Infect. Dis. 4:35 (1982); Collins, W.P., In: Alternative Immunoassays, John Wiley & Sons, NY (1985); Ngo, T.T. et al., In: Enzyme Mediated Immunoassay, Plenum Press, NY (1985); incorporated by reference herein.

In one embodiment, the affinity purified monospecific antibodies ALZab2, based on the sequences in table 1b that we have made can be used in any immuno assay test system to detect ALZASp, in dot blot methods or in quantitative methods using sandwich ELISA and trap ELISA techniques as outlined in figures 5 A+B.

In another embodiment the presence of, alzas, and mRNA in a cell or in the fluid as is described herein can be determined by any means capable of detecting mRNA encoding these proteins.

Such nucleic acid based assays may use either DNA or RNA to detect dsas, alzas alzas1 and alzas2 mRNA. In one embodiment, the assays may be performed on RNA that has been extracted from blood cells as described in the specifications herein. The assays may be done in situ on biopsied tissue using for example PCR (Mullis, K.B., Cold Spring Harbour Symp. Quant. Biol - 51:263-273 (1986); Saiki, R.K., et al., Bio/Technology 3:1008-1012 (1985); Mullis K. et al., U.S. Patent 4,683,202; Erlich, H., U.S. Patent 4,582,788; Saiki, R. et al., US 4,683,194 and Mullis, K.B., et al., Met. Enzymol. 155:335-350 (1987), transcription-based amplification systems (Kwoh D et al., Proc. Natl. Acad. Sci. (U.S.A.) 86:1173 (1989); Gingeras TR et al., PCT appl. WC 88/10315; Davey, C. et al. European Patent Application Publication no. 329,822), etc.

### B. Prognostic Uses

The present invention additionally provides a capacity to predict very early whether an individual has Alzheimer's disease. Thus, any of the above-described assays may be performed on an asymptomatic individual in order to assess the restaging of these diseases.

### C. Therapeutic Uses

Significantly, the present invention provides a means for treating AD. Such treatment may be either "prophylactic" or "therapeutic." A prophylactic treatment is one that is provided in advance of any clinical symptom of AD in order to prevent or attenuate any subsequent onset of the disease. A therapeutic treatment is one that is provided in response to the onset of a symptom of AD and serves to attenuate an actual symptom of the disease.

In one embodiment, such treatment is provided by administering to a patient in need of such treatment an effective amount of an antibody, or an antibody fragment (F(ab'), F(ab')2, single chain antibodies, etc.) that is capable of binding to, ALZASp (aa 67-79).

The immunotherapy can be used in the form of an active vaccine, e.g. multiple antigenic epitopes based on the ALZAS sequence, or of a passive vaccine, e.g. humanized antibodies (see humanized antibodies in the following references which are incorporated by reference (Morrison, S.L., Science, 229:1202-1207 (1985); Oi, V.T. et al., BioTechniques 4:214 (1986); Jones, U.T. et al., Nature 321:552-525 (1986); Verhoeyan et al., Science 239:1534 (1988).

In another embodiment the desired therapy may be obtained by targeting the nucleic acid molecules, specifically the promoter sequences Palzas 1-7 of the present invention with "antisense" nucleic acid molecules. Antisense oligonucleotides are disclosed in European Patent Application Publication Nos. 263,740; 335,451; and 329,882, and in PCT Publication No. WO90/00624. Used herein, an aniti-sense nucleotide is a DNA or RNA whose sequence is complementary to the sequence of 6 or PALZ 1 to PALZ 7 described herein, such that it is capable of binding to, or hybridizing with, an endogenous promoter or heat shock sequence in a manner sufficient to impair its transcription, and significantly inactivate it in a cell; or whose sequence is complementary to alzas - nucleotide 96-135, or alzas1 - nucleotide 55-156, or alzas2 - nucleotide 96-135 and thereby impair (i.e. attenuate or prevent) it's the translation into protein. These molecules may be transported into the cell using the. Protein-Polycation Conjugates system (Beug, H. et al. United States patent 5,354,844 11/10 1994) in an appropriate pharmaceutical compound, also see Oldham, R.K. (In: Principles of Biotherapy, Raven Press, NA, 1987), and Ledley, F.D., In: Biotechnology, A Comprehensive Treatise, volume 7B, Gene Technology, VCH Publishers, Inc. NY, pp399/458 (1989)). The principles of the present invention can be used to provide a prophylactic gene therapy to individuals who, due to inherited genetic mutations, or somatic cell mutation, are predisposed to Alzheimer's disease.

Thus, in one embodiment of this invention, an antisense oligonucleotide that is designed to specifically block transcription of translation of a alzas, alzas1 or alzas2, mRNA transcript can be used to impair the expression of ALZASp, in a cell, and thereby provide a treatment for AD.

In yet another embodiment such treatment is provided by administering to the patient an effective amount of a cys-cys reducing reagent which will inhibit or reduce the oligomerization of ALZASp.

### III. Administration of the Molecules of the Present Invention

Additional pharmaceutical methods may be employed to control the duration of action of any of the aforementioned reagents used to treat AD. Such techniques are disclosed in Remingtons's Pharmaceutical Sciences (1980).

### PUZZLING QUESTIONS ANSWERED BY THIS INVENTION

The discovery that the genes which encode Aβ might be separate from APP mRNA has provided insight into how, and why, mutations at different regions of the APP gene, including regions outside the Aβ encoding region, cause AD. It has also provided an explanation for the AD like symptoms produced in transgenic animals expressing APP mRNA, and in mice in which APP mRNA transcription was blocked.

This invention has permitted us to construct a comphrensive model of AD.

### THE MODEL.

Internally, in response to adherence of certain infectious agents or secretory products of such agents to localized areas on cell membranes, specific cytoskeleton proteins become highly phosphorylated by activated tyrosine phosphatases and form organised cytoskeleton structures. The latter interacts with the cell membrane directly below the point of contact on the surface membrane and this facilitate entry of the agent into the cytoplasm. Tau may be specifically mobilized and hyperphosphorylated in response to ALZAS interacting with membranes of specific sub sets of neurons. Externally, the entry of ALZAS into neuronal plasma membrane causes the release and binding of IgG, IgM and other immunological factors to the membrane bound protein, particularly the unique c-terminal sequence. This acts as a signal for the initiation of the complement cascade which targets cells bearing ALZAS for destruction and attempted removal.

Based on our results, the above considerations and results reported by others in the literature, we have proposed the following model for AD which shows how the various AD related proteins interact to produce the AD phenotype. The model can be rigorously tested.

### STAGING OF AD

1. ALZAS transcription and expression is activated by any of a number of external and intracellular factors including stress, viral and other pathogenic invasions and environmental toxins, in a specific cell type (perhaps lymphocytes). ALZAS molecules aggregate to remain soluble, traverse the cytoplasm using the same route as APP and are secreted.
2. Secreted ALZAS, because of the unique c-terminal which is expressed from intron sequences is treated as foreign (i.e. an endogenous pathogen). Soluble antibody IgM, IgG, IgA (anti-ALZAS Ig), produced in response to ALZAS bind to and neutralize ALZAS.
3. Conditions that decrease the efficiency of the immune system e.g. certain viral infections, physiological aging etc, permit an increasing number of ALZAS molecules to escape destruction. These molecules interact with cell membranes of specific sub populations of neurons (target cells) and enter and damage the membrane.
4. Interaction of ALZAS with target neuron membranes triggers two major physiological responses. (i) Activation of tyrosine phosphatases which convert tau to PHF-tau. The latter interacts with the cytoplasmic side of the membrane below the points of interaction with ALZAS and form long neurofibrillary structures which facilitate entry of ALZAS into the cytoplasm and (ii), Activation of the complement system which attempts to destroy ALZAS. Because the specific amino acid sequences targeted by complement protease are identical in ALZAS and APP, APP is also cut by the ALZAS directed protease/s. The complement destruction of neuronal membrane APP results in a local acute inflammatory reaction and autoimmune-type inflammatory destruction of the affected neurons.
5. ALZAS interacts with ApoE proteins in a way that hides the hydrophobic domain and allows the complex to remain soluble in the cytoplasm; in this manner ALZAS is chaperoned (transported in a soluble form) between the neuronal cytoplasm the ER and the neuronal plasma membrane. If ALZAS oligomerize with ApoE2, and to a lesser extent ApoE3, Cys-Cys interaction occurs between the two molecules which tends to maintain ALZAS in a soluble form in which the protein cannot traverse the ER.
6. If ALZAS oligomerize with ApoE4 which does not have a Cys residue, on contact with the ER membranes ALZAS regains the monomeric state in which the hydrophobic membrane signal is exposed and the protein competes with, replaces and prevents APP from occupying sites on the ER. normally ER bound APP remains in the cytoplasm where it is degraded by cellular proteolytic activity into smaller fragments including various Aβ species.
7. PS1/PS2 form part of a highly specific APP binding site in neuronal ER membranes. Mutations in any part of the PS1/2 molecule which have the slightest effect on conformation or mutations in APP which occur close to the transmembrane signal or within it considerably diminishes the specificity of interaction between APP and the PS1/PS2 component of the binding site and confers an even higher preference for ALZAS binding to the APP transmembrane site. Whereas APP is translocated and carries out all its essential biochemical activities, ALZAS probably causes dissolution of the membrane which results in loss of function of a number of ER membrane proteins.
8. Aβ which is perhaps the most protease resistant portion of the APP protein, and elongated, sticky, neurofibrils produced from PHF-tau which form tangles, are released into intracellular spaces as the neuronal membranes "dissolve".

Having now generally described the invention, through references and examples that makes it more readily understood by any one sufficiently skilled in the art, it must be pointed out that these are not intended to be limiting of the present invention, unless specified.

At present using ALZab2 in the immunoblot procedure described earlier, we are detecting ALZASp in blood samples taken from one out of five (5 of 19) people who were undergoing cognitive and other test given to people having signs that are suspected to be associated with the onset of AD. The detection was done on 1µl whole blood taken from specimens which were freshly drawn from the patients for other routine determinations associated with the institution's AD test routine. Using the same procedure as above, the antibody detects ALZASp in 100% of the blood specimens taken from AD victims very soon after death.

This invention is defined by the claims.

### Figure 4

A = description of antigen trap ELISA test
B = description of antibody trap ELISA test
C-E Detection of expression of ALZAS related proteins ALZASp (ALZAS), in 2µl of human blood dotted on nylon membranes using chemiluminescent detection on system. For dot blots, proteins extracted from brain and lymphocyte by ammonium sulphate precipitation were dialysed and boiled for 5 minutes in 5% SDS. 2µl of the SDS treated proteins were spotted onto nylon membranes, treated with the appropriate antibody and detected with chemiluminescent system:
(F) Dot blots of serum from 28 autopsy confirmed AD victims was probed with ALZab2 (at least 50% of these patients we incorrectly diagnosed for a condition other than AD before death) ;D 6 & 7 = positive control; D 9 & 10 = negative control
(G) Dot blots of serum from suspected early AD patients, patients with depression and autopsy confirmed AD victims: A 1= physiological aging; B 1 = Parkinsons disease, A 2-4, 6-7, B 3, 5, C 2, 3 = AD with depression; A 5, 8, B 2 3, 5, 7, 8, 9, 10 = suspected AD, C 1, 6-10 = serum from confirmed AD; D 1-10 serum from normal humans.
(H) Detection of endogenous anti-ALZAS IgG in patients blood serum and saliva: (i) serum samples from Japanese patients who died in hospital in 1995; filled circles = serum from AD patients open circles = autopsy confirmed humans without AD; (ii) serum from patients diagnosed as AD and clinically normal people selected at random: filled circles = AD patients; empty circles = clinically normal persons; (iii) serum from people with M > L related FAD, open circles = family members without the mutation without clinical symptoms AD; filled circles = family members with the mutation with no clinical signs of AD; large circle with filled interior family member with the mutation and with clinical AD.
(I) western blots; proteins in ammonium sulphate precipitates were electrophoresed on 17.5 % acrylamide gels, blotted onto nylon filters, reacted with the appropriate antibody and detected with the immuno-chemiluminescent system, 2. proteins were isolated from AD hippocampus and treated with antibody ALZab2; 3. proteins were isolated from AD hippocampus and treated with ALZab3.

### Figure 5A-D

(A) Isolation of ALZAS from cortex obtained from a single autopsy confirmed AD patient. The protein was affinity purified on anti ALZab2 antibody linked to CNBR-sepharose. The gel was blotted onto nylon a nylon membrane and the protein detected with a chemiluminescent detection system.
(B) Silver stain of SDS gel: proteins obtained following affinity purification of ALZAS from serum from a late stage AD patient. In addition to ALZAS, ALZAS bound to various immunoglobulins and to APOE was detected on the silver stained gel and confirmed by specific staining duplicate gels
(C) Detection of ALZAS-IgG complexes present on gel of 5B with anti-human IgG (Fc fragment).
(D) Western blot of cationic non SDS polyacrylamide gel following electrophoresis of serum from (a) a patient with sporadic AD, and (b) a patient with swedish mutation AD. The gels were reacted first with anti ALZASab1 and then with the chemiluminescence system. ALZAS was detected in a and ALZAS2 in b. The upper band in a is ALZAS and in b is ALZAS2 both complexed to human IgG. ALZAS and ALZAS2 have slightly different isoelectric points which can be seen on the gels.

**TABLE 1a**

| PCR primers used to detect mRNA in post mortem brains and lymphocytes. |
|---|
| Primers: |
| |
| |

**Table 1b**

| Antibodies prepared against the following epitopes were used to detect proteins from post mortem brains and lymphocytes. | |
|---|---|
| ALZab2 | |
| ALZab5 | EVGKLDC |

| | |
|---|---|
| * = Cys residue added to facilitate conjugation KLH. | |

## Claims

1. A nucleic acid molecule free from natural contaminants selected from the group consisting of alzas promoters and alzas the sequence of which is SEQ ID NO:11 SEQ ID NO:17, SEQ ID NO:18, SEQ ID NO:19, SEQ ID NO:20, SEQ ID NO:21, SEQ ID NO:22, SEQ ID NO:23 respectively.

2. A protein molecule free from natural contaminants consisting of ALZAS the sequence of which is SEQ ID NO: 12, particularly amino acids 68-79

3. An antibody specific for ALZAS the sequence of ALZAS being SEQ ID NO:12 , particularly whereas the antibody is directed against amino acid 68-79 (ALZab2 described in table 1b).

4. An antibody or an antibody fragment, or single chain antibodies, or a combination thereof, capable of binding specifically to amino acid 68-79 of SEQ ID NO:12 (ALZab2 described in Table 1) for use in the prophylactic or therapeutic treatment of Alzheimer's disease.

5. Use of an antibody, or an antibody fragment or of a single chain antibody or a combination thereof, capable of binding specifically to amino acid 68-79 of SEQ ID NO:12 (ALZab2 described in Table 1) (see claims 2 or 3) for the manufacture of a medicament for a prophylactic treatment of Alzheimer's disease.

6. An antibody as defined in claim 4 wherein the antibody is a monospecific polyclonal antibody or a monoclonal antibody or a recombinant antibody or a humanized antibody,

7. Antisense nucleotide sequences specific for the molecule having the sequence complementary to alzas promoter SEQ ID NO:17, SEQ ID NO:18, SEQ ID NO:19, SEQ ID NO:20, SEQ ID N0:21, SEQ ID NO:22, SEQ ID NO:23.

8. In vitro use of an antisense nucleotide sequence of claim 7.

9. A method for the diagnosis or the presymptomatic diagnosis of Alzheimer's disease comprising detecting in a sample of tissue or a fluid or a protein as defined by SEQ ID NO: 12 (amino acid 68-79) or comprising of detecting alzas mRNA as defined by SEQ ID NO:11.

10. A method for the diagnosis or presymptomatic diagnosis of Alzheimer's comprising detecting in a fluid sample an antibody specific for amino acids 68-79 (SEQ ID NO:12) (see also claims 2 and 3).

11. An antibody as defined in claim 4 or 6 wherein the disease is Alzheimer's disease

12. The use as defined in claim 5 wherein the antibody is as defined in claim 6.

13. The use as defined in claim 12 wherein the disease is as defined in claim 11.

## Patentansprüche

1. Ein Nukleinsäuremolekül welches frei von natürlichen Kontaminationen ist und aus der Gruppe ausgewählt ist, die aus dem alzas Promotor und alzas besteht, deren Sequenz SEQ ID NR:11, SEQ ID NR: 17, SEQ ID NR: 18, SEQ ID NR: 19, SEQ ID NR:20, SEQ ID NR:21, SEQ ID NR:22, SEQ ID NR:23 ist.

2. Ein Proteinmolekül welches frei von natürlichen Kontaminationen ist und aus ALZAS besteht, dessen Sequenz SEQ ID NR: 12, insbesondere Aminosäuren 68-79 ist.

3. Ein für ALZAS spezifischer Antikörper, wobei die Sequenz von ALZAS SEQ ID NR:12 ist und der Antikörper insbesondere gegen Aminosäuren 68-79 (ALZab2 beschrieben in Tabelle 1b) gerichtet ist.

4. Ein Antikörper oder ein Antikörperfragment oder Einzelkettenantikörper, oder eine Kombination aus diesen, die spezifisch an Aminosäuren 68-79 der SEQ ID NR:12 (ALZab2 beschrieben in Tabelle 1b) binden können, zum Gebrauch in der prophylaktischen oder therapeutischen Behandlung der Alzheimer Krankheit.

5. Der Gebrauch eines Antikörpers oder eines Antikörperfragments oder eines Einzelkettenantikörpers, oder einer Kombination aus diesen, die spezifisch an Aminosäuren 68-79 der SEQ ID NR:12 (ALZab2 beschrieben in Tabelle 1b) binden können (s. Ansprüche 2 oder 3) zur Herstellung eines Medikaments für die prophylaktische Behandlung der Alzheimer Krankheit.

6. Ein wie in Anspruch 4 definierter Antikörper, wobei der Antikörper ein monospezifischer polyklonaler Antikörper oder ein monoklonaler Antikörper oder ein rekombinanter Antikörper oder ein humanisierter Antikörper ist.

7. Antisense-Nukleotidsequenzen, die spezifisch für das Molekül sind, welches die zum alzas Promotor SEQ ID NR:17, SEQ ID NR:18, SEQ ID NR:19, SEQ ID NR:20, SEQ ID NR:21, SEQ ID NR:22, SEQ ID NR:23 komplementäre Sequenz besitzt.

8. Der in vitro Gebrauch einer Antisense-Nukleotidsequenz von Anspruch 7.

9. Eine Methode zur Diagnose oder präsymptomatischen Diagnose der Alzheimer Krankheit, welche den Nachweis eines Proteins definiert durch SEQ ID NR:12 (Aminosäuren 68-79) in einer Gewebeprobe oder einer Flüssigkeit, oder den Nachweis von alzas mRNA wie durch SEQ ID NR:11 definiert beinhaltet.

10. Eine Methode zur Diagnose oder präsymptomatischen Diagnose der Alzheimer Krankheit, welche den Nachweis eines Antikörpers mit Spezifität für Aminosäuren 68-79 (SEQ ID NR:12) (s.a. Ansprüche 2 und 3) beinhaltet.

11. Ein Antikörper wie definiert in den Ansprüchen 4 oder 6, wobei die Krankheit Alzheimer Krankheit ist.

12. Der Gebrauch wie definiert in Anspruch 5 wobei der Antikörper wie in Anspruch 6 definiert ist.

13. Der Gebrauch wie definiert in Anspruch 11, wobei die Krankheit wie definiert in Anspruch 11 ist.

## Revendications

1. Une molécule d'acide nucléique sans contaminant naturel faisant partie du groupe constitué d'ALZAS et des promoteurs de ALZAS, dont les séquences sont, respectivement, SEQ ID NO : 11, SEQ ID NO: 17, SEQ ID NO : 18, SEQ ID NO : 19, SEQ ID NO : 20, SEQ ID NO : 21, SEQ ID NO : 22 et SEQ ID NO : 23.

2. Une protéine sans contaminant naturel constituée d'ALZAS (dont la séquence est SEQ ID NO : 12) et plus particulièrement des acides aminés 68-79.

3. Un anticorps spécifique de ALZAS (dont la séquence est SEQ ID NO : 12) lorsque cet anticorps est dirigé contre un acide aminé 68-79 (ALZab2 décrit dans le tableau 1b).

4. Un anticorps, un fragment d'anticorps, des anticorps simple chaîne ou une combinaison de ceux-ci capables de se lier spécifiquement à un acide aminé 68-79 de la séquence SEQ ID NO : 12 (ALZab2 décrit dans le tableau 1b) afin de traiter la maladie d'Alzheimer de façon prophylactique ou thérapeutique.

5. Un anticorps, un fragment d'anticorps, des anticorps simple chaîne ou une combinaison de ceux-ci capables de se lier spécifiquement à un acide aminé 68-79 de la séquence SEQ ID NO : 12 (ALZab2 décrit dans le tableau 1b) (voir les revendications 2 et 3) afin de fabriquer un médicament permettant le traitement prophylactique de la maladie d'Alzheimer.

6. Un anticorps tel que défini en 4 lorsqu'il s'agit d'un anticorps polyclonal monospécifique, d'un anticorps monoclonal, d'un anticorps recombinant ou d'un anticorps humanisé.

7. Séquences nucléotides antisens spécifiques de la molécule ayant la séquence complémentaire aux promoteurs d'ALZAS dont les séquences sont SEQ ID NO : 17, SEQ ID NO : 18, SEQ ID NO : 19, SEQ ID NO : 20, SEQ ID NO : 21, SEQ ID NO : 22 et SEQ ID NO : 23.

8. L'utilisation *in vitro* d'une des séquences nucléotides antisens annoncées dans la revendication 7.

9. Une méthode de diagnostic ou de diagnostic présymptomatique de la maladie d'Alzheimer comprenant sa détection dans un échantillon de tissu, un liquide biologique ou une protéine définie par la SEQ ID NO : 12 (acide aminé 68-79), ou bien la détection de l'ARNm de ALZAS tel que défini par la séquence SEQ ID NO : 11.

10. Une méthode de diagnostic ou de diagnostic présymptomatique de la maladie d'Alzheimer comprenant la détection dans un échantillon de liquide biologique d'un anticorps spécifique des acides aminés 68-79 (SEQ ID NO : 12) (voir également les revendications 2 et 3).

11. Un anticorps tel que revendiqué en 4 ou en 6 lorsque la maladie est la maladie d'Alzheimer.

12. L'utilisation revendiquée en 5 lorsque l'anticorps est celui revendiqué en 6.

13. L'utilisation revendiquée en 12 lorsque la maladie est celle revendiquée en 11.
